# FASCICULE DE BREVET EUROPEEN

(11) **EP 2 183 364 B1**
(45) Date de publication et mention de la délivrance du brevet: **22.05.2013**
(21) Numéro de dépôt: 08829271.9
(22) Date de dépôt: 28.08.2008
(51) Int. Cl.: C12N 15/10

(54) **PROCEDE DE SELECTION DE LEVURES OENOLOGIQUES**
VERFAHREN ZUR AUSWAHL VON WEINHEFEN
METHOD FOR SELECTING OENOLOGICAL YEASTS

(30) Priorité: 29.08.2007 FR 0757245
(43) Date de publication de la demande: 12.05.2010
(73) Titulaire: Societe d'Applications de Recherches et de Conseils Oenologiques (SARCO), 33100 Bordeaux (FR)
(72) Inventeur: MARULLO, Philippe, F-33370 Tresses (FR)
(74) Mandataire: Lebrette, Camille
(86) Numéro de dépôt international: PCT/FR2008/051540
(87) Numéro de publication internationale: WO 2009/030863

(56) Documents cités:
- MARULLO PHILIPPE ET AL: "Single QTL mapping and nucleotide-level resolution of a physiologic trait in wine Saccharomyces cerevisiae strains" FEMS YEAST RESEARCH, vol. 7, no. 6, septembre 2007 (2007-09), pages 941-952, XP002472533 ISSN: 1567-1356 cité dans la demande
- SINCLAIR K ET AL: "The ASP1 gene of Saccharomyces cerevisiae, encoding the intracellular isozyme of L-asparaginase." GENE 24 JUN 1994, vol. 144, no. 1, 24 juin 1994 (1994-06-24), pages 37-43, XP002472534 ISSN: 0378-1119
- CLAUSEN ET AL: "PAD1 encodes phenylacrylic acid decarboxylase which confers resistance to cinnaic acid in Saccharomyces cerevisiae" GENE, ELSEVIER, AMSTERDAM, NL, vol. 142, 1994, pages 107-112, XP002139270 ISSN: 0378-1119
- SMIT A ET AL: "Enhancing volatile phenol concentrations in wine by expressing various phenolic acid decarboxylase genes in Saccharomyces cerevisiae" JOURNAL OF AGRICULTURAL AND FOOD CHEMISTRY, AMERICAN CHEMICAL SOCIETY, WASHINGTON, US, vol. 51, no. 17, 13 août 2003 (2003-08-13), pages 4909-4915, XP002428316 ISSN: 0021-8561
- SHINOHARA T ET AL: "Distribution of phenolic yeasts and production of phenolic off-flavors in wine fermentation." JOURNAL OF BIOSCIENCE AND BIOENGINEERING 2000, vol. 90, no. 1, 2000, pages 90-97, XP002523087 ISSN: 1389-1723
- MARULLO PHILIPPE ET AL: "Breeding strategies for combining fermentative qualities and reducing off-flavor production in a wine yeast model" FEMS YEAST RESEARCH, vol. 6, no. 2, mars 2006 (2006-03), pages 268-279, XP002523088 ISSN: 1567-1356

## Description

### Domaine technique

L'invention concerne un procédé de sélection de levures oenologiques de l'espèce *Saccharomyces cerevisiae* destinées à être utilisées en agroalimentaire. Plus précisément, l'invention concerne un procédé permettant de sélectionner de manière rapide et sûre des levures en fonction de caractères particuliers recherchés. L'invention propose plus particulièrement de sélectionner des levures oenologiques en fonction d'un caractère oenologique lié à la production de phénols volatiles, et le cas échéant d'autres caractères oenologiques particuliers, tels que les capacités fermentaires des levures et/ou les propriétés olfactives, gustatives, visuelles, de conservation etc. que l'on souhaite conférer à un vin.

### Etat de la technique

Les levures entrent dans de nombreux processus d'élaboration de produits alimentaires destinés à la consommation animale, humaine et non humaine. Par exemple, des levures fermentaires sont utilisées pour la fabrication du pain, de la bière, du vin etc. Il est désormais connu d'utiliser des levures particulières pour élaborer de tels produits alimentaires, de manière à influer sur le processus de fabrication et/ou sur les propriétés organoleptiques du produit fini.

En ce qui concerne plus particulièrement le vin, l'arôme du vin est d'une grande complexité en raison des constituants volatils qui le composent. Au cours des différentes étapes biochimiques et technologiques de la vinification, le moût de raisin, à la saveur relativement neutre, se transforme en une boisson alcoolisée extrêmement riche sur le plan organoleptique. Les levures fermentaires telles que les levures de l'espèce *Saccharomyces cerevisiae,* ont un impact non négligeable sur la formation de la flaveur du vin. Au cours de la fermentation alcoolique, trois catégories de molécules odorantes sont produites par la levure : les arômes fermentaires, les défauts olfactifs et les arômes variétaux.

Les arômes fermentaires sont des molécules issues du métabolisme des sucres et des acides aminés. Elles participent à la note vineuse générale.

Les défauts olfactifs sont notamment liés à la production de sulfure d'hydrogène, qui présente une odeur nauséabonde d'oeuf pourri. Sa production résulte de la réduction des sulfates et des sulfites présents dans le moût de raisin, et implique le complexe enzymatique de la sulfite réductase. Il est connu, pour éviter la surproduction de sulfure d'hydrogène, de différer de quelques jours la date de sulfitage après la fermentation alcoolique.

Les défauts olfactifs sont également liés à la surproduction d'acides volatils tels que l'acide acétique, qui s'accumulent dans le vin au cours des différentes étapes de vinification.

Les arômes variétaux, spécifiques aux cépages sont également responsables de la typicité du vin. Certains d'entre eux, appartenant à la catégorie des thiols, sont révélés par les levures à partir de précurseurs inodores et non volatils contenus dans le moût. Il a été démontré que certaines souches de levures révélaient mieux que d'autres le potentiel aromatique contenu dans le moût de raisin.

Les performances fermentaires des levures oenologiques, c'est-à-dire leurs aptitudes à achever rapidement une fermentation alcoolique, sont dépendantes de trois facteurs principaux : l'assimilation du substrat azoté, l'assimilation des sucres fermentescibles et la tolérance à l'éthanol.

Les levures oenologiques présentent une variabilité génétique importante entraînant une forte variabilité métabolique influençant ces différents paramètres lors du procédé de vinification. Cette forte variabilité s'explique par un degré élevé de polymorphisme génétique. Le plus souvent, les caractères technologiques des levures se répartissent de manière quantitative au sein d'une population. Cette distribution traduit l'influence de plusieurs régions du génome (locus) comprenant un ou plusieurs gènes d'intérêt. Au sein d'une population, ces gènes présentent des variations de séquences (dites allèles) dont certaines apportent un avantage technologique particulier. Le plus souvent ces régions génétiques ne sont que grossièrement localisées, elles sont couramment appelées QTL, pour « Quantitative Trait Loci » (Lynch & Walsh 1998).

Actuellement, il est connu de sélectionner des levures oenologiques en fonction de leurs caractères oenologiques, ou aptitudes technologiques liées au vin, par criblage phénotypique de collections de levures. Cette méthodologie présente comme principal défaut la nécessité d'effectuer des tests technologiques sur d'importantes populations. Cette démarche ralentit fortement les délais de sélection et par conséquence la taille de la population testée. Lorsque plusieurs caractères technologiques sont désirés, on réalise classiquement des programmes de croisement et on sélectionne les meilleurs individus. Selon le degré de complexité des programmes de croisement (hybridation, ségrégation de F1, backcross...) et le nombre de paramètres à maitriser, la taille des échantillons à tester génère très vite des programmes de sélection trop longs et trop couteux. De plus, il n'est pas possible avec cette méthode de sectionner a priori des levures oenologiques comportant certains allèles favorables déjà connus intervenant dans un ou plusieurs caractères oenologiques.

### Exposé de l'invention

Aussi, dans l'invention, on cherche à fournir une méthode de sélection rapide et efficace de levures oenologiques de l'espèce *Saccharomyces cerevisiae* pouvant présenter un caractère oenologique lié à la production de phénols volatiles, et le cas échéant également un ou plusieurs caractères oenologiques d'intérêt différents. Dans l'invention, on cherche également à obtenir une souche de levures présentant un fonds génétique intéressant d'un point de vu vinicole et un allèle favorable intervenant pour un caractère oenologique spécifique lié à la production de phénols volatiles et dont la souche de levures est initialement dépourvue.

Pour cela, l'invention propose de procéder à une sélection des levures oenologiques de l'espèce *Saccharomyces cerevisiae* présentant ledit caractère oenologique intéressant préalablement associé à un locus spécifique, à l'aide de marqueurs moléculaires aptes à identifier un allèle favorable dudit locus. Un marqueur moléculaire est un point du génome présentant des polymorphismes de séquence (allèles) entre plusieurs individus. Des techniques de typage moléculaire permettent d'identifier cet allèle et de connaitre rapidement le génotype de nombreux individus. Les marqueurs moléculaires peuvent être obtenus à partir de multiples méthodes telles que la PCR/RFLP, DHLPC, hybridation de sondes spécifiques marquées, les microsatellites, les puces à ADN etc. D'une manière générale, on entend par typage moléculaire toute méthode de biologie moléculaire apte à détecter une variation de séquence ADN et/ou ARN et/ou peptidique entre deux individus. Les marqueurs moléculaires en question peuvent soit identifier directement la variation de séquence responsable du caractère oenologique, soit être localisés à une position voisine de celle-ci.

Selon l'invention, on peut également introduire par introgression dans un fonds génétique choisi, un locus associé à un caractère oenologique d'intérêt, lié à la production de phénols volatiles, dont un allèle favorable provient d'une souche donneuse, et procéder à une sélection des descendants présentant ledit allèle favorable à l'aide de marqueurs moléculaires. L'introgression consiste en une introduction progressive d'au moins un locus d'intérêt d'une souche de levure donneuse dans le génome d'une autre souche de levure par une succession d'hybridations et de croisements en retour.

L'invention a donc pour objet un procédé de sélection de levures oenologiques de l'espèce *Saccharomyces cerevisiae,* tel que défini dans la revendication 1. Dans des modes de mise en oeuvre particuliers, le procédé répond à l'une ou plusieurs des caractéristiques des revendications 2 à 11.

Par sélection, on entend l'identification et l'isolement des levures oenologiques c'est-à-dire des levures intervenant dans le processus de fabrication du vin, notamment dans le processus de fermentation. Dans le domaine de l'oenologie, on utilise principalement les levures du genre *Saccharomyces* et en particulier de l'espèce *Saccharomyces cerevisiae.*

Par caractère oenologique, on entend aussi bien un caractère lié aux propriétés organoleptiques finales du vin qu'un caractère intervenant dans le processus de fabrication du vin. Le caractère oenologique d'intérêt peut ainsi être lié aux arômes fermentaires ou variétaux, aux défauts olfactifs tels que la surproduction de sulfure d'hydrogène, ou d'acidité volatile ; mais aussi des paramètres fermentaires telle que la tolérance à l'éthanol qui peut être évaluée en mesurant la quantité de sucres résiduels en fin de fermentation alcoolique (FA), ou bien la vitesse de fermentation qui peut être évaluée en mesurant le temps nécessaire à une souche pour dégager 80% de CO₂ productible.

On entend par allèle favorable une variation nucléotidique de séquence située à un locus donné qui différentie des souches de levures au sein d'une même espèce. Lorsque cet allèle est présent le caractère oenologique associé est amélioré.

On entend par marqueur moléculaire une variation nucléotidique de séquence identifiée par des méthodes de biologie moléculaire qui renseigne sur la présence d'un allèle favorable. Ce marqueur peut soit être l'allèle favorable en question soit être physiquement et génétiquement lié à celui-ci.

D'une manière générale, un QTL est identifié à partir de la corrélation existant entre un locus marqueur et le paramètre quantitatif étudié, ici un caractère oenologique. Si le marqueur est lié à un paramètre quantitatif, il y a une différence statistiquement significative entre les valeurs phénotypiques des différentes souches de levures présentant un génotype différent au locus en question.

Dans l'invention, on a plus particulièrement identifié sept allèles favorables associés chacun à au moins un caractère oenologique particulièrement intéressant, à savoir :
- l'allèle *ASP1*,* ayant pour séquence SEQ ID N°1, qui influe de manière avantageuse sur la vitesse relative à mi fermentation, la durée de fermentation et la teneur en sucres résiduels en fin de fermentation ; les levures ainsi sélectionnées ont des performances améliorées pour ces caractères, en ce sens que la vitesse relative à mi-fermentation est supérieure à 0,60 et de préférence supérieure à 0,70, la durée de fermentation est inférieure à 120 heures et de préférence inférieure à 105 heures et la teneur en sucres résiduels est inférieure à 7 g/L et de préférence inférieure à 2 g/L.
- l'allèle *YOL083w*,* ayant pour séquence SEQ ID N°4, qui réduit de manière avantageuse la production de sulfure d'hydrogène (H₂S) ;
- l'allèle *PAD1*,* ayant pour séquence SEQ ID N°7, qui influe de manière avantageuse sur la production de phénol volatile. Plus précisément, les levures porteuses de l'allèle *PAD1** ne produisent pas de phénol volatil tels que le 4-vinylphénol et 2-methoxy-4-vinylphenol ;
- l'allèle *VEL1*,* ayant pour séquence SEQ ID N 10, et l'allèle *WSC3** ayant pour séquence SEQ ID N 13 ; qui influent de manière avantageuse sur la durée de fermentation en réduisant le temps nécessaire pour dégager 80% de CO2 productible;
- l'allèle *YOR385w*,* ayant pour séquence SEQ ID N 19, et l'allèle *NUD1*,* ayant pour séquence SEQ ID N 16, qui influent tous deux de manière avantageuse sur la teneur en sucres résiduels en fin de fermentation alcoolique et en particulier lors des fermentations réalisées à de fortes températures (supérieures à 28°C).

Dans tous les cas, l'étape de sélection après introgression se fait à l'aide de marqueurs moléculaires associés à l'allèle favorable.

D'une manière générale, on procède de la manière suivante :
- on sélectionne une souche de levures dite « élite » ;
- on sélectionne à l'aide de marqueurs moléculaires une souche de levures donneuses présentant au moins un allèle favorable associé au caractère oenologique d'intérêt ;
- on introduit par introgression l'allèle favorable dans des descendants de la souche élite ;
- on sélectionne à l'aide de marqueurs moléculaires des descendants de la souche élite présentant l'allèle favorable.

La souche élite est une souche de levures présentant un fonds génétique intéressant. Généralement, une souche de levures élite est une variété de référence très adaptée à la production de vin et dont seuls quelques caractères doivent être améliorés sans changer le comportement général de la souche. En suivant à l'aide de marqueurs moléculaires les allèles des levures donneuses et élites, il est possible en quelques croisements d'obtenir des levures quasiment isogéniques aux levures élites mais ayant intégré un ou plusieurs locus des levures donneuses.

Selon des exemples de réalisation de l'invention, il est possible d'obtenir des souches de levures présentant l'allèle sous forme homozygote ou hétérozygote.

L'invention concerne également une séquence nucléotidique ayant la séquence nucléotidique de SEQ ID N°7.

Un autre aspect de l'invention est une souche de levures de l'espèce *Saccharomyces cerevisiae* obtenue par un procédé tel que défini dans la revendication 4, ne produisant pas de phénols volatiles.

### Brève description des figures

Figure 1 : représentation de la valeur phénotypique des descendants de l'hybride F1 en fonction de l'hérédité au locus *ASP1* pour les caractères oenologiques V50/Vmax, durée de fermentation et quantité de sucres résiduels en fin de FA ;
Figure 2 : représentation de la valeur phénotypique des descendants de l'hybride F1 en fonction de l'hérédité au locus *YOL083* pour le caractère oenologique relatif à la production d'H2S ;
Figure 3 : typage PCR/RFLP d'une collection de souches de levures oenologiques au locus *ASP1 ;*
Figure 4 : représentation schématique des étapes du procédé d'introgression assisté par marqueurs moléculaires de l'invention;
Figure 5 : carte des marqueurs d'une souche de levures obtenue par le procédé de sélection selon l'invention ;
Figure 6 : représentation schématique de la construction de l'hybride H4 nécessaire à la cartographie de QTL par backcross. Ici les descendants sont sélectionnés à partir de leur valeur phénotypique pour le caractère oenologique relatif à la quantité de sucres résiduels en fin de fermentation alcoolique ;

- Figure 7 : représentation de la répartition des souches obtenues lors des étapes d'introgression selon la figure 6, en fonction de la quantité de sucres résiduels en fin de fermentation ;
- Figure 8 : représentation schématique de la valeur phénotypique de 42 descendants de l'hybride H4 obtenu selon la figure 6, pour le caractère oenologique relatif à la quantité de sucres résiduels en fin de fermentation alcoolique ; A étant le phénotype du parent A, B le phénotype du parent B, 8d le phénotype de huit descendants H4 génotypés sur puce à ADN et 34d le phénotype des 34 autres descendants H4 ;
- Figure 9 : carte des marqueurs obtenus des huit descendants de H4 issus du retrocroisement entre les levures A et B de la figure 8, et sélectionnés pour leur faible quantité de sucres résiduels en fin de fermentation alcoolique ; cette carte montre la répartition des marqueurs SNP issus du parent A et du parent B ;
- Figures 10A à 10D : représentation schématique de la répartition de la population en fonction de l'hérédité (parent A ou B) aux locus *NUD1* (figure 10A), *VEL1* (figure 10B), *WSC3* (figure 10C) et YOR385 (figure 10D).

### Description détaillée de l'invention

L'invention est présentée plus en détails dans les exemples non limitatifs qui suivent.

Les exemples ci-dessous décrivent plus particulièrement sept locus particuliers intervenant sur plusieurs caractères oenologiques intéressants, étant bien entendu que le procédé de sélection selon l'invention peut s'appliquer de manière analogique à d'autres locus et caractères oenologiques associés.

### 1- Obtention de locus d'intérêt par cartographie QTL

D'une manière générale, l'identification d'un locus et des marqueurs moléculaires correspondants se déroule en trois étapes principales, à savoir le croisement de deux souches parentales phénotypiquement différentes pour le caractère oenologique étudié ; l'analyse physiologique et génétique de la descendance avec mesure physiologique et typage moléculaire ; la cartographie QTL par analyse de liaison (Marullo et al, 2007).

### A] Exemple 1

Plus particulièrement, dans un premier exemple décrit ici, deux souches de levures oenologiques parentales SB et GN telles que décrites dans Marullo et al, 2007 et qui sont différentes pour un grand nombre de paramètres technologiques sont croisées afin de générer un hybride F1. Une cinquantaine d'individus descendant de cet hybride F1 sont génotypés à l'aide de puces à ADN afin d'identifier sur l'ensemble de leur génome des marqueurs bi-alléliques différenciant SB et GN.

D'une manière générale, un test d'association entre l'hérédité des marqueurs et des valeurs de mesures technologiques permet, de manière connue, d'identifier des locus associés à ces caractères (Lander et Bolstein, 1989). En utilisant un jeu de données phénotypiques obtenu sur ces descendants décrit dans Marullo et al 2006, on établit des corrélations statistiques entre la présence de certains marqueurs définis par les puces à ADN et des paramètres technologiques.

L'aptitude d'une centaine de clones monosporiques à achever une fermentation difficile a été mesurée sur un milieu MS300 contenant 300g/L de sucres (Marullo et al. 2004).

A partir de ces clones, différents paramètres ont été analysés.

La tolérance à l'éthanol est généralement estimée en mesurant la teneur en alcool (%vol) à la fin de la fermentation alcoolique (Romano et al. 1985). La tolérance à l'éthanol s'entend de la concentration maximale d'alcool que peut produire une souche en présence d'un excès de sucre. Avec les souches oenologiques, la tolérance à l'éthanol varie entre 12 et 17% vol suivant les fonds génétiques. La tolérance à l'éthanol est aussi évaluée en termes de quantité de sucres résiduels en fin de fermentation alcoolique dans ce même milieu. Dans ce cas plus une souche laisse de sucres résiduels moins elle est tolérante (Marullo et al 2004). Dans le cas de cette étude c'est ce paramètre qui est mesuré.

Le rapport V50/Vmax et la durée de fermentation sont mesurés à partir de fermentations réalisées sur milieu KP 210 g/L de sucres (Marullo et al. 2006). Le Vmax est la vitesse maximale de dégagement de CO₂. Le V50/Vmax correspond à la vitesse de production de CO₂ à mi-fermentation (50% de CO2 dégagé) divisé par le Vmax. Ce paramètre traduit la chute de vitesse se produisant après le Vmax. C'est un indicateur de l'activité fermentaire pendant la phase stationnaire de croissance qui constitue plus de deux tiers de la réaction. La durée de fermentation est le temps nécessaire pour consommer la totalité des sucres sans tenir compte de la durée de la phase de latence.

La production de vinyl phénol (caractère POF) est mesurée après fermentation dans un milieu complet comportant de l'acide paracoumarique (Marullo et al. 2006, Chatonnet et al. 1993). La sélection de souches POF-est particulièrement avantageuse pour la fermentation de vins blancs secs. En effet, il est connu qu'au cours de la vinification des vins blancs, la levure peut être responsable de la production de phénols volatils du type 4-vinylphénol et 2-methoxy-4-vinylphenol synthétisés à partir des acides phénoliques contenus dans le raisin. Ces arômes volatils indésirables laissent percevoir au dégustateur un manque de finesse générale avec des notes de « gouache » ou « médicament ». Les souches aptes à révéler ce type d'arômes sont nommées [POF+] pour Phenolic Off Flavours.

La production de sulfure d'hydrogène (H₂S) est mesurée à partir d'un test Biggy réalisé sur boite de Pétri. Ce test permet d'évaluer visuellement le noircissement de colonies de levures après 3 jours de culture sur le milieu. Plus une souche produit de sulfure d'hydrogène (H₂S) plus sa couleur est sombre. On utilise une échelle visuelle allant de 1 (clair), pour faible production, à 5 (sombre), pour forte production (Romano et al. 1985).

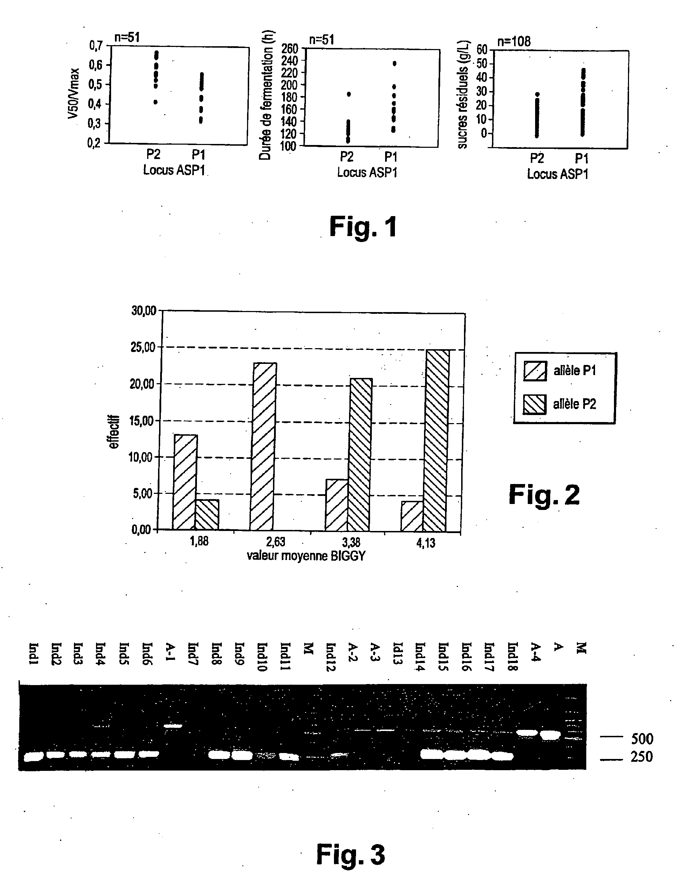

Sur les figures 1 et 2, on peut voir notamment la répartition des effectifs pour un phénotype donné, à savoir respectivement les caractères de V50/Vmax, durée de fermentation et tolérance à l'éthanol (figure 1), et la production d' H₂S (figure 2), en fonction de leur génotype au locus d'intérêt.

Plus précisément, sur la figure1, on peut voir la répartition d'une population de levures en fonction de l'hérédité au locus *ASP1* pour les caractères de V50/Vmax, durée de fermentation et tolérance à l'éthanol. Les locus P2 et P1 représentent respectivement l'hérédité des parents SB et GN. Le nombre (n) d'individus phénotypés est indiqué en haut de chaque graphique. Le locus revendiqué *ASP1** présente par rapport à la séquence sauvage *(wt)* la substitution D142H consistant en la substitution d'une asparagine par de l'aspartate en position 142 de la séquence primaire en aminoacide. Pour les trois caractères oenologiques en question, il existe une différence significative entre les deux sous populations. Cette différence est mesurée grâce à un test statistique non paramétrique mesurant la répartition des individus entre les deux sous populations (voir Tableau 1).

Sur la figure 2, on peut voir la répartition d'une population de levures en fonction de l'hérédité au locus *YOL083* pour le caractère H₂S. Les individus ayant hérité de l'allèle de la souche parentale GN présentent une faible production de sulfure d'hydrogène (faible valeur sur l'échelle Biggy). L'allèle issu de la souche parentale GN (désigné P1 sur la figure) est donc considéré comme l'allèle favorable *YOL083w*.* Là encore, on constate des différences de valeurs phénotypiques entre les deux sous populations, chacune d'elles comportant n=106 individus.

Le tableau 1 ci-dessous indique le nom et le positionnement de différents locus ainsi que la valeur de corrélation statistique avec le paramètre technologique auquel ils sont liés. Le test non paramétrique de Mann-Whitney indiqué dans le tableau 1 est notamment décrit par Berry & Lindgren (1995).

**Tableau 1 : Liaison statistique entre les locus cartographiés et les caractères d'intérêt.**

| Locus (gène) | Caractère associé | Probabilité de liaison (-log P) Test Mann-Whitney | Nombre de descendants testés |
|---|---|---|---|
| *ASP1* | Sucres résiduels en fin de fermentation alcoolique sur un milieu MS300 | 4,51 | 108 |
| *ASP1* | V50/Vmax | 3,31 | 51 |
| *ASP1* | Temps de fermentation | 2,54 | 51 |
| *YOL083w* | Production d'H₂S | 9,42 | 106 |
| *PAD1* | Production de phénols volatils | 7,34 (100% de concordance) | 51 |

### B] Exemple 2

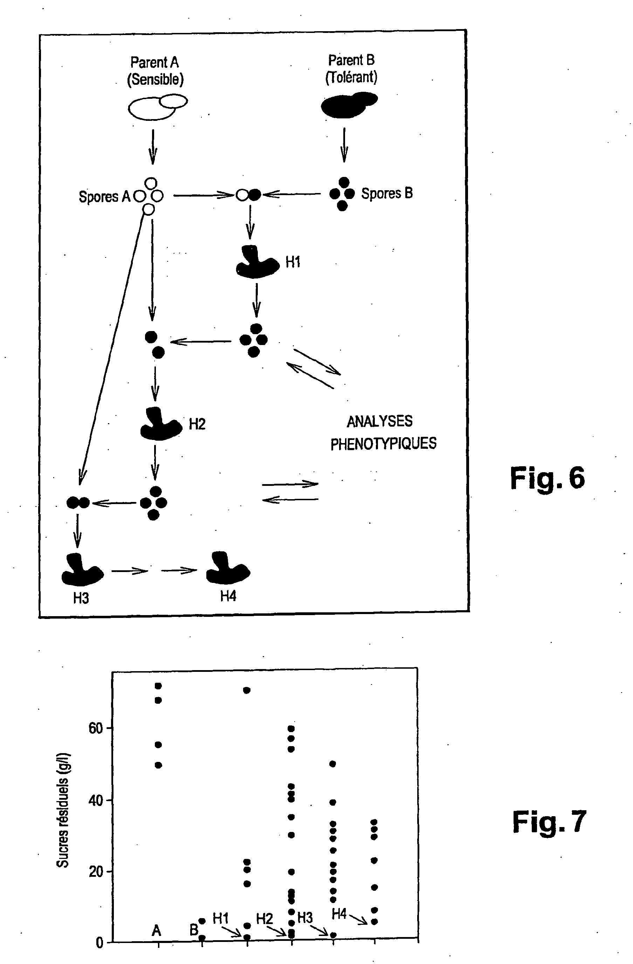

Dans un autre exemple de cartographie de QTL réalisée à partir d'un programme de backcross (figure 6), on a cartographié plusieurs locus d'intérêt conférant un avantage pour des paramètres d'achèvement de la fermentation à forte température (28°C).

En génétique quantitative, l'analyse de liaison peut être menée à partir des valeurs phénotypiques de la descendance d'un hybride F1. Il est également possible d'analyser une population NIL (« nearly isogenic line », c'est-à-dire « lignée isogénique proche »). Cette stratégie réduit le nombre de caractères étudiés mais augmente considérablement la puissance de détection des QTL. Dans le cas de notre travail, cette population a été obtenue par back cross assisté par analyses phénotypiques à partir de deux souches commerciales, l'une sensible A et l'autre tolérante souche B (décrite par ailleurs dans l'exemple 1 comme la souche SB) à une fermentation conduite à 28°C (Marullo et al. 2007b) (Figure 6).

Statistiquement, 6,25% du génome de l'hybride H4 (Back cross de 4ème génération) vient du parent B. La variation des valeurs phénotypiques observée chez les descendants de cet hybride H4 est en théorie expliquée par la ségrégation de ces 6,25%. Autrement dit ces quelques pourcentages de génome contiennent les QTL majoritairement impliqués dans la différence entre les parents A et B.

L'étude de cartographie réalisée a été menée sur 42 descendants de l'hybride H4 issus de 11 tétrades.

Dans un premier temps, le phénotype de chacun des 42 descendants de H4 a été établi en réalisant des fermentations alcooliques à 28°C selon les conditions décrites dans Marullo et al 2007b, dans un milieu synthétique contenant 260g/L de sucres et carencé en ergostérol.

A la fin de la fermentation deux paramètres ont été évalués :
- le temps nécessaire pour dégager 80% du CO₂ productible (t80, en heures) ; Plus ce temps est faible et plus la souche présente une fermentation rapide et donc souhaitable ; et
- la teneur en sucres résiduels en fin de fermentation alcoolique à 28°C (SR en fin de FA en g/L).

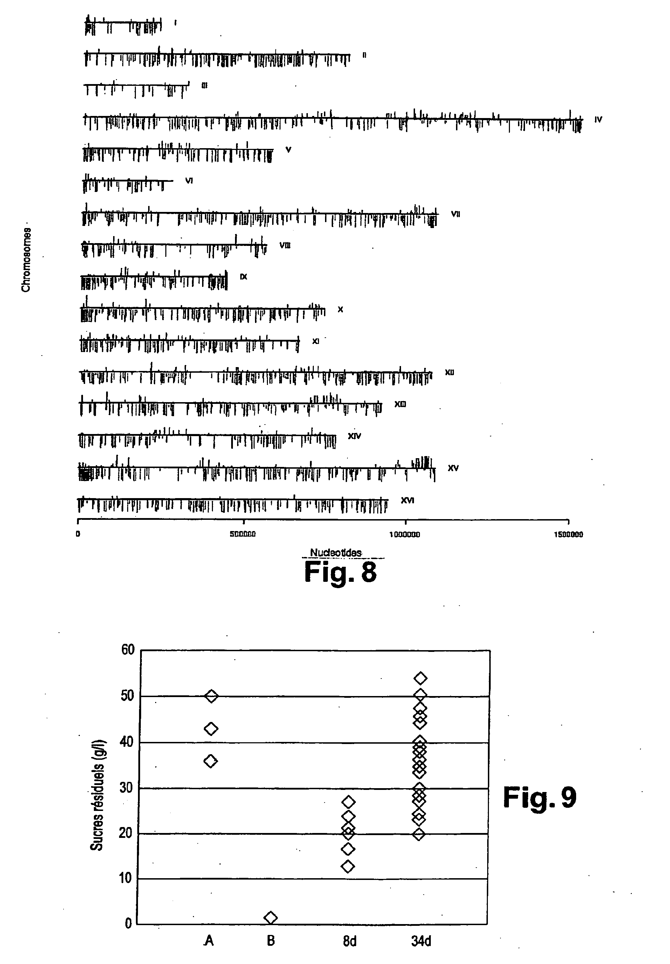

La figure 9 montre la répartition des valeurs de sucres résiduels en fin de fermentation alcoolique pour les 42 descendants et pour les deux souches parentales A et B. Parmi les 42 descendants, 8 on été sélectionnés qui présentent les phénotypes les plus intéressants.

Dans un second temps des marqueurs moléculaires caractérisant ces descendants on été recherchés. Afin d'obtenir rapidement un grand nombre de marqueurs, des SNP entre les souches A et B ont été recherchés grâce à des puces à ADN Affymétrix© de type YTM. Ces SNP ont été recherchés sur la base des différences de profils d'hybridation entre les souches A et B selon une procédure similaire à celle décrite par Gresham et al. 2006.

Dans le but de concentrer la recherche de QTL sur la part de génome de la souche B introgressée au cours des backcross, les huit descendants de l'hybride H4 présentant des phénotypes « optimaux» ont été génotypés et les SNP spécifiques aux parents B ont été recherchés dans leur génome.

Sur les 2960 marqueurs identifiés entre A et B, 316 sont retrouvés au moins chez 4 des 8 descendants ayant un phénotype optimisé.

La figure 8 montre l'hérédité des allèles des parents A et B pour les 8 descendants de l'hybride H4 présentant un phénotype d'intérêt (peu de sucres résiduels en fin de fermentation alcoolique à 28°C). Les allèles représentés ont été retrouvés chez au moins deux descendants.

En considérant que l'espace entre deux chromosomes est égal à 1, chaque trait est proportionnel à son occurrence dans les puces à ADN (de 0,2 à 0,8). Les allèles sont rangés par chromosomes. Les pics (traits verticaux) situés en dessous des chromosomes (traits horizontaux) représentent les allèles hérités de A. Les pics situés au dessus des chromosomes représentent les allèles hérités de B.

Parmi cet ensemble de 316 marqueurs, un jeu de 70 marqueurs bialléliques différenciant potentiellement A et B a été sélectionné, afin de réaliser une cartographie de QTL. Le polymorphisme des marqueurs bialléliques choisis a été suivi par CE-SSCP (Capillary electrophorises single strand chain polymorphism) chez les 42 individus. Grâce à cette méthode mise en place par Kozlowski et Krzyzosiak, il est possible de suivre le polymorphisme des différents individus de manière rapide (480 échantillons par série) et reproductible (matériel automatisé).

Une fois les valeurs phénotypiques et les génotypes des différents individus obtenus, une corrélation entre la présence d'un marqueur et la valeur d'un phénotype a été recherchée au moyen de tests statistiques selon des méthodes standards d'analyse de liaison (Test de Mann-Whitney selon Berry & Lindgren (1995)).

Le tableau 2 ci-dessous indique le nom et le positionnement de différents locus ainsi que la valeur de corrélation statistique avec le paramètre technologique auquel ils sont liés.

**Tableau 2 : Liaison statistique entre les locus cartographiés et les caractères d'intérêt.**

| Locus (gène) | Probabilité de liaison (-log P) Test Mann-Whitney | Caractère associé | Nombre de descendants testés |
|---|---|---|---|
| *NUD1* | 2,3268 | Sucres résiduels en fin de fermentation alcoolique à 28°C | 42 |
| *YOR385w* | 2,43 | Sucres résiduels en fin de fermentation alcoolique à 28°C | 42 |
| *WSC3* | 2,1 | temps pour dégager 80% du CO₂ | 42 |
| *VEL1* | 2,017 | temps pour dégager 80% du CO₂ | 42 |

Les allèles issus de la souche B sont considérés comme favorables, car ils entraînent une diminution du temps pour dégager 80% de CO₂ et de la quantité de sucres résiduels en fin de fermentation alcoolique à 28°C.

### 2- Utilisation de marqueurs moléculaires de type PCR/RFLP pour traquer les locus d'intérêt

On dessine des amorces PCR permettant d'amplifier des petites portions génomiques proches des pics de déséquilibre de liaison précédemment identifiés.

Avantageusement, lesdites séquences sont choisies de manière à présenter un polymorphisme de séquence détectable par RFLP et pouvoir ainsi être facilement suivies, sans avoir recourt à des puces à ADN. L'utilisation d'un enzyme de restriction approprié permet de distinguer l'allèle du parent GN (ou A) de l'allèle du parent SB (ou B) (Tableau 3).

Dans le cas où aucun site RFLP n'est trouvé, il est bien entendu possible d'utiliser d'autres méthodes de typage comme le séquençage ou l'utilisation d'amorces dégénérées ou bien le suivi de l'allèle en question par EC-SSCP.

**Tableau 3 : Corrélation entre l'allèle favorable d'un locus donné et l'amorce PCR permettant la mise en évidence dudit allèle favorable**

| Caractère | Nom du locus | Amorce pour PCR | Parent porteur de l'allèle favorable | Enzyme coupant l'allèle favorable | Profil SSCP |
|---|---|---|---|---|---|
| Production H2S | YOL083 | P118, P119 | GN | Nla3 | |
| SR en fin de FA sur un milieu MS300 | ASP1 | P124 P125 | SB | Hin4I | |
| Vitesse de fermentation | ASP1 | P124 P125 | SB | Hin4l | |
| Phase de latence | YOL083 | P118 P119 | GN | NIa3 | |
| Vitesse à mi fermentation | ASP1 | P124 P125 | SB | Hin4l | |
| Production de vinylphénol | PAD1 | P400 P401 | GN | BgI1 | |
| t80 | WSC3 | P386 p387 | SB | | XV_114 |
| t80 | VEL1 | P428 P429 | SB | | VII_10 |
| SR en fin de FA à 28°C | NUD1 | P388 P389 | SB | Taq1 | XV_1036 |
| SR en fin de FA à 28°C | YOR385W | P486 p487 | SB | | XV_1064 |

Le tableau 4 ci-dessous récapitule les effets des QTL *NUD1 WSC3 VEL1* et YOR385w sur la variation totale du phénotype observé chez la descendance de l'hybride H4 de l'exemple 2.

**Tableau 4 : Effets de QTL sur la variation phénotypique totale de la descendance de H4**

| | part de variance expliquée | ANOVA pvalue associée | phénotype |
|---|---|---|---|
| NUD1 | 18% | 0,40% | SR |
| WSC3 | 7% | 3% | t80 |
| VEL1 | 14% | 0,80% | t80 |
| YOR385w | 25% | 0,05% | SR |

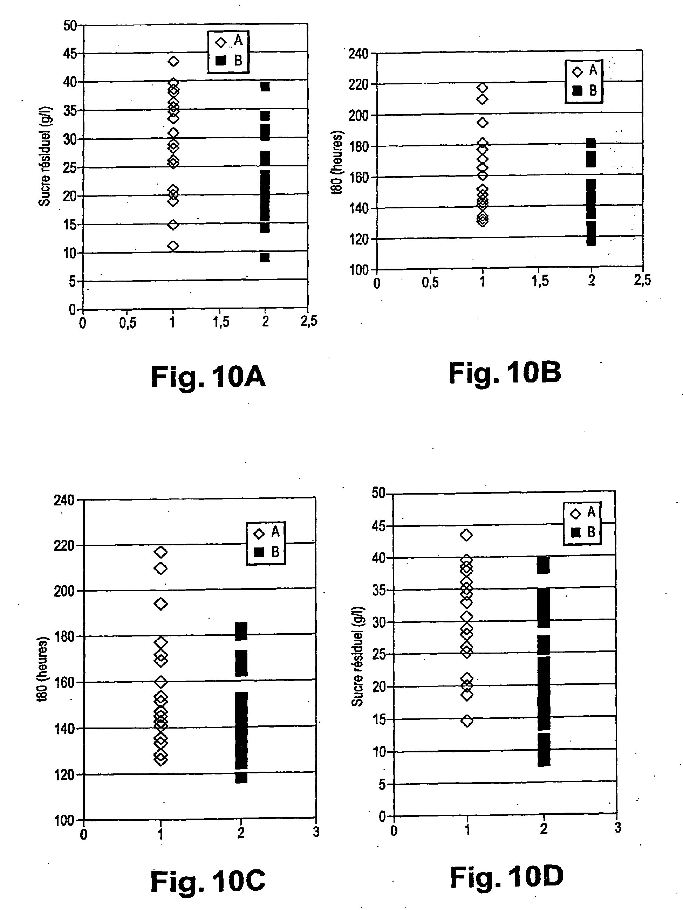

Les figures 10A, 10B, 10C et 10D montrent respectivement la répartition des phénotypes des descendants selon leur hérédité pour l'allèle en question.

### 3- Résultats

### Allèle ASP1*

La présence de l'allèle *ASP1** permet une amélioration de trois paramètres technologiques clé en oenologie. En effet, l'allèle *ASP1** est statistiquement corrélé à plusieurs paramètres oenologiques d'intérêt tel que la vitesse relative à mi fermentation (V50/Vmax), la durée de fermentation et la teneur en sucres résiduelss. Dans le fonds génétique utilisé pour la cartographie génétique, (c'est-à-dire la descendance de l'hybride F1 issu du croisement GN x SB) ces paramètres sont modifiés respectivement de +14% -12% et -50% chez les individus porteurs de l'allèle *ASP1** par rapport aux individus non porteurs.

L'allèle *ASP1^{*},* porté par la souche SB, est défini par la séquence SEQ ID N°1 amplifiée à l'aide des amorces P124 de séquence SEQ ID N°2 et P125 de séquence SEQ ID N°3.

Selon la base de données SGD (Saccharomyce Genome Database - www.yeastgenome.org-), ce couple d'amorces amplifie le chromosome IV entre les bases 1108932 et 1109749.

L'allèle d'intérêt *ASP1** porte notamment une mutation de guanine en cytosine sur la base 424 du gène *ASP1,* lui faisant perdre le site de coupure de l'enzyme Hin4I. Il est ainsi aisé d'identifier la présence de l'allèle *ASP1** dans une souche de levures par typage RFLP, la présence dudit allèle étant caractérisée par l'absence de coupure par l'enzyme Hin4l du fragment de 817 pb amplifié par les amorces de séquences SEQ ID N°2 et SEQ ID N°3.

L'allèle *ASP1** a été recherché chez une vingtaine de souches industrielles par typage. Comme cela est visible sur la figure 3, représentant un typage RFLP d'une vingtaine de souches oenologiques au locus *ASP1,* seule la souche industrielle Ind19 et quatre de ces descendants (Ind19-1, Ind19-2, Ind19-3 et SB) portent cette mutation sur une vingtaine de fonds génétiques analysés. Dans ce cas la fréquence allèlique de cet allèle est de 0.25%

### Allèle YOL083w*

La présence de l'allèle *YOL083w** permet une diminution significative de la production de sulfure d'hydrogène mesuré à l'aide du test de référence Biggy (Romano et al. 1985). Cet allèle, porté par la souche GN, est très significativement corrélé à ce paramètre (figure 2). Dans le fonds génétique utilisé pour la cartographie génétique (c'est-à-dire la descendance de l'hybride F1 issu du croisement GN x SB) ce paramètre est diminué de deux points, soit de 4 à 2, sur l'échelle BIGGY qui en comporte cinq.

L'allèle *YOL083** est défini par la séquence SEQ ID N°4 amplifiée à l'aide des amorces p118 de séquence SEQ ID N°5 et p119 de séquence SEQ ID N°6.

Selon la base de données SGD ce couple d'amorces amplifie le chromosome XV entre les bases 166278 et 166844.

L'allèle d'intérêt *YOL083** porte notamment une mutation sur la base 894 du gène *YOL083w* de la souche GN de cytosine en arginine, qui lui fait perdre le site de coupure de l'enzyme NIa III.

Il est ainsi aisé d'identifier la présence de l'allèle *YOL083** dans une souche de levures par typage RFLP, la présence dudit allèle étant caractérisée par l'absence de coupure par l'enzyme NIa III du fragment de 566 pb amplifié par les amorces de séquence SEQ ID N°5 et SEQ ID N°6.

### Allèle PAD1*

La présence de l'allèle *PAD1** permet l'expression du caractère POF-qui annule la production de phénols volatiles, à savoir les vinyl-4 phénol et vinyl 4-guaiacol, à partir de précurseurs tels que l'acide paracoumarique (Chatonnet et al. 1993). Cet allèle porté par la souche GN est entièrement corrélé à ce paramètre (tableau 1). L'analyse de la séquence de l'allèle *PAD1** de la souche GN révèle un polymorphisme de séquence par rapport à la souche SB à la position 638 du gène *PAD1* (Goodey & Tubb, 1982). Cette modification entraîne la conversion de l'acide aspartique en glycine en position 213 de la protéine. Cet acide aspartique est fortement conservé chez d'autres espèces et pourrait être impliqué dans l'activité catalytique de l'enzyme d'après l'analyse des séquences protéiques issues du génome de plusieurs organismes procaryotes et eucaryotes tels que GI17932869, GI56553716, GI75235662, GI68475725, et GI42520414. Les codes GI permettent d'avoir accès aux séquences sur le site www.ncbi.nlm.nih.gov.

L'allèle *PAD1** est défini par la séquence SEQ ID N°7 amplifiée à l'aide des amorces p400 de séquence SEQ ID N°8 et p401 de séquence SEQ ID N°9.

Selon la base de données SGD, ce couple d'amorces amplifie le chromosome IV entre les bases 1510790 et 1511668.

L'allèle d'intérêt *PAD1** porte notamment une mutation de l'adénine en guanine sur la base 638 du gène *PAD1* chez la souche donneuse GN qui fait apparaître un site de coupure de l'enzyme *Bg*/I.

Il est ainsi aisé d'identifier la présence de l'allèle *PAD1** dans une souche de levures par typage RFLP, la présence dudit allèle étant caractérisée par la présence de coupure par l'enzyme *Bg*/I du fragment de 813 pb amplifié par les amorces de séquence SEQ ID N°8 et SEQ ID N°9.

### Allèle VEL1*

La présence de l'allèle *VEL1** permet une diminution du temps nécessaire pour dégager 80% de CO₂ productible (t80).

Cet allèle, situé sur le chromosome VII de *Saccharomyces cerevisiae* et porté par la souche B, est très significativement lié à ce paramètre.

L'allèle *VEL1** est défini par la séquence SEQ ID N°10, qui peut être obtenue par séquençage à partir des amorces p428 (SEQ ID N°11) et p429 (SEQ ID N°12). Il est ainsi aisé d'identifier la présence de l'allèle *VEL1** dans une souche de levure par séquençage à partir de ces amorces.

Cet allèle *VEL1** est situé entre les bases 11060 et 11105 du chromosome VII. L'allèle d'intérêt *VEL1** porte une mutation en position 11061 de cytosine en guanine, et une mutation en position 11064 de cytosine en tyrosine par rapport à la souche de référence S288c (http://www.yeastgenome.org/). Cet allèle n'est retrouvé chez aucune des 34 souches séquencées par le centre Sanger (http://www.sanger.ac.uk/) il présente donc une fréquence allèlique de 8.10⁻⁴.

### Allèle WSC3*

La présence de l'allèle *WSC3** permet une diminution du temps nécessaire pour dégager 80% de CO2 productible (t80).

Cet allèle, situé sur le chromosome XV chez *Saccharomyces cerevisiae* et porté par la souche B, est significativement lié à ce paramètre.

L'allèle *WSC3** est défini par la séquence SEQ ID N°13 obtenue par séquençage à partir des amorces p386 (SEQ ID N°14) et p387 (SEQ ID N°15). Il est ainsi aisé d'identifier la présence de l'allèle *WSC3** dans une souche de levure par séquençage à partir de ces amorces.

Cet allèle est situé entre la base 114491 et la base 114544 du chromosome XV.

L'allèle d'intérêt *WSC3** porte une mutation en position 1144531 de guanine en cytosine par rapport à la souche de référence S288c (http://www.yeastgenome.org/).

Cet allèle n'est retrouvé chez aucune des 34 souches séquencées par le centre Sanger (http://www.sanger.ac.uk/) il présente donc une fréquence allèlique de 8.10⁻⁴.

### Allèle NUD1*

La présence de l'allèle *NUD1** permet une diminution de la teneur en sucres résiduels en fin de fermentation alcoolique à 28°C

Cet allèle, situé sur le chromosome XV chez *Saccharomyces cerevisiae* et porté par la souche B, est très significativement lié à ce paramètre.

L'allèle d'intérêt *NUD1** est défini par la séquence SEQ ID N°16 obtenue par séquençage à partir des amorces p388 (SEQ ID N°17) et p389 (SEQ ID N°18). Cet allèle est situé entre la base 1036809 et la base 1036868 du chromosome XV.

L'allèle d'intérêt *NUD1** porte une mutation en position 1036827 de cytosine en tyrosine par rapport à la souche de référence S288c (http://www.yeastgenome.org/).

Cet allèle est retrouvé avec une fréquence allèlique de 5% environ dans le cas présent (sur les 34 souches de levures *Saccharomyces cerevisiae* séquencées par le centre Sanger (http://www.sanger.ac.uk/), 8 présentées cet allèle *NUD1*).*

Il est aisé d'identifier la présence de l'allèle *NUD1** dans une souche de levure par typage RFLP, la présence dudit allèle étant caractérisé par la présence d'un site de coupure supplémentaire par l'enzyme Taq1, en position 118 du fragment de 400 pb obtenu par les amorces de séquences SEQ ID N°17 et SEQ ID N°18.

### Allèle YOR385w*

La présence de l'allèle *YOR385w** permet une diminution de la teneur en sucres résiduels en fin de fermentation alcoolique à 28°C

Cet allèle, situé sur le chromosome XV chez *Saccharomyces cerevisiae* et porté par la souche B, est très significativement lié à ce paramètre.

L'allèle d'intérêt *YOR385w** est défini par la séquence SEQ ID N°19 obtenue par séquençage à partir des amorces p486 (SEQ ID N°20) et p487 (SEQ ID N°21). Cet allèle est situé entre la base 1064543 et la base 1064598 du chromosome XV.

L'allèle d'intérêt *YOR385w** porte une mutation en position en position 1064572 de tyrosine en guanine par rapport à la souche de référence S288c (http://www,yeastgenome.org/).

Cet allèle est retrouvé chez 6 des 34 souches de levures *Saccharomyces cerevisiae* séquencées par le centre Sanger (http://www.sanger.ac.uk/), et présente donc une fréquence allèlique de 3% environ

Il est aisé d'identifier la présence de l'allèle *YOR385w** dans une souche de levure par séquençage à partir des amorces de séquences SEQ ID N°20 et SEQ ID N°21.

### 4- Exemples détaillés de mise en oeuvre du procédé de sélection selon l'invention

### Exemple 1 : Obtention de souches de levures Saccharomyces Cerevisiae intégrant les allèles ASP1*, YOL083* et PAD1*

On cherche à fabriquer et sélectionner une souche de levures oenologiques hybrides présentant de bonnes capacités fermentaires et exempt de défauts aromatiques en utilisant, conformément au procédé de l'invention, des marqueurs moléculaires.

Plus précisément, on cherche à améliorer la souche SB, ou souche élite, par introgression assistée par marqueur moléculaire en utilisant comme souche donneuse la souche GN, de manière à introduire les allèles *YOL083** et *PAD1** et à conserver l'allèle *ASP1** qui confère les aptitudes fermentaires, initialement présent dans la souche SB.

A chaque cycle d'introgression, la présence des l'allèles d'intérêt *YOL083*, PAD1** et *ASP1** est vérifiée par test moléculaire. De manière classique, la présence de ces allèles est vérifiée en amplifiant à l'aide d'une réaction de PCR l'ADN génomique de chaque descendant à tester avec les couples d'amorces déjà cité p124/p125, p118/p119 et p400/p401. Le protocole d'amplification est celui classiquement utilisé par l'homme de l'art. Une fois ces fragments amplifiés, la présence de l'allèle favorable est recherchée en soumettant ces fragments PCR à une digestion enzymatique avec les enzymes décrites plus haut. Cette procédure PCR/RFLP permet d'identifier les individus ayant hérité de l'allèle d'intérêt et donc technologiquement plus performant en s'affranchissant des tests phénotypiques.

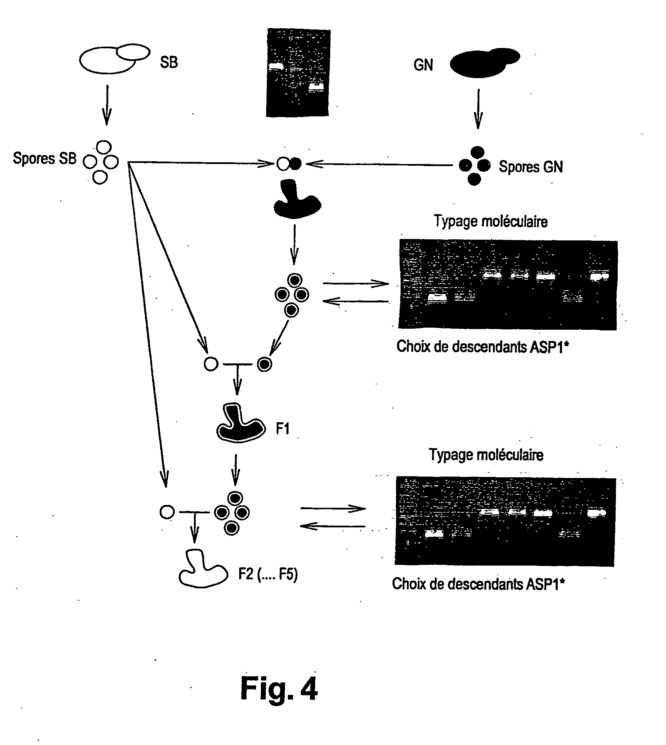

Les étapes d'introgression sont représentées de manière schématique à la figure 4 pour le locus *ASP1,* suivi à l'aide du marqueur *ASP1** définissant l'allèle favorable à conserver. Les allèles favorables des locus *YOL083* et *PAD1* peuvent être suivis de manière similaire.

Comme cela est représenté à la figure 4, à partir de l'hybride F1 un descendant portant l'allèle *ASP1** est sélectionné par typage moléculaire. Ce descendant est croisé avec la souche SB. Après 5 cycles de rétrocroisement basé sur le même principe on obtient la souche F5 et ses descendants. La souche F5 et ses descendants présentent un génome très proche de la souche SB d'origine, environ 96% d'identité, à l'exception des locus introgréssés.

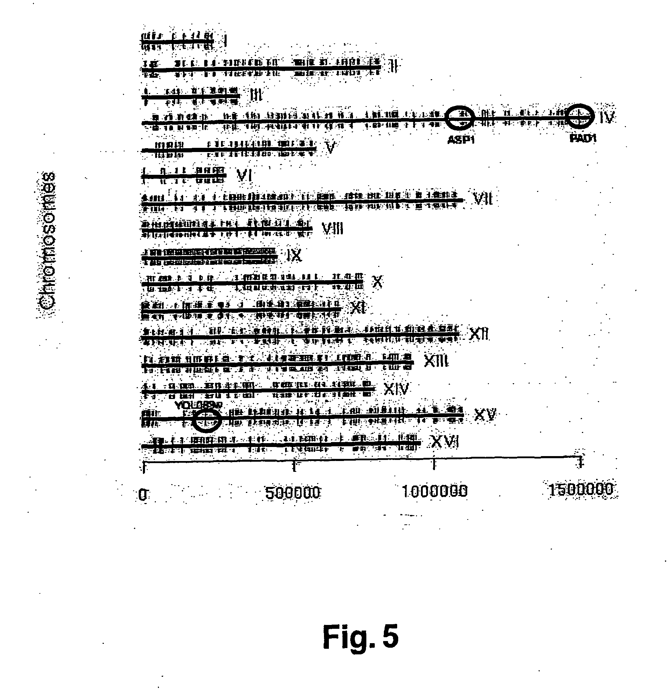

Après cinq cycles de croisement on obtient un descendant de l'hybride F5 la souche F5-1 D, dont la carte génétique est représentée à la figure 5.

Le degré de parenté entre la souche F5-1 D et la souche SB est mesuré en utilisant une puce à ADN (Affymetrix) selon un protocole d'hybridation et une procédure de typage moléculaire telle que décrite dans Marullo et al. 2007a.

La figure 5 montre que plus de 94% des marqueurs sont issus de la souche SB et en particulier l'allèle *ASP1*.* Parmi les marqueurs issus de la souche GN on retrouve en particulier les allèles *YOL083** et *PAD1** qui ont été introduits durant la procédure par le même mode opératoire.

### Exemple 2 : Obtention de souches de levures Saccharomyces Cerevisiae intégrant les allèles ASP1*, WSC3*, NUD1*, VEL1* et YOR385w*

Les allèles *ASP1* WSC3*, NUD1*, VEL1* et *YOR385w** ont été introduits dans des souches d'intérêt par introgression assistée par marqueur moléculaire selon le même procédé que celui décrit dans la figure 4. A chaque fois, la présence de l'allèle est vérifiée par le test moléculaire ce qui permet de s'affranchir des tests technologiques.

La souche X est ici étudiée plus spécifiquement. Cette souche a été obtenue en croisant la souche industrielle CLIB 2014 et la souche SB porteuse des allèles *ASP1*, WSC3*, NUD1*, VEL1* et *YOR385w*.* Une descendance issue de l'hybride de ce croisement a été typée pour la présence des l'allèles *ASP1*, WSC3*, NUD1*, VEL1* et YOR385w*.*

Un de ces descendants, porteur desdits allèles d'intérêt, a été à nouveau croisé avec la souche CLIB 2014 pour donner un hybride de seconde génération. L'expérience a été répétée 4 fois selon le même schéma que montré en exemple 1.

A la fin on obtient la souche X génétiquement proche de la souche CLIB 2014 (congénique à plus de 93%) et porteuse des l'allèles *ASP1*, WSC3*, NUD1*, VEL1** et *YOR385w*.*

Par rapport à la souche CLIB 2014 de départ, ladite souche X présente par exemple un taux de sucres résiduels bien plus faible dans deux conditions de vinification (voir tableau 5 ci-dessous).

Les souches CLIB 2014 et X sont évaluées à deux températures 24°C et 28°C sur le milieu KP contenant 260 g/L de sucres fermentescibles.

Le tableau 5 ci-dessous montre les performances des souches CLIB 2014 et X à 24°C et 28°C. Pour chaque condition, la souche X fermente mieux que la souche CLIB 2014

**Tableau 5: Comparatif entre les souches CLIB2014 et X pour le pourcentage d'éthanol produit et la quantité de sucres résiduels en fin de fermentation alcoolique à 24°C et 28°C.**

| Conditions | 24°C CLIB 2014 | 28°C CLIB 2014 | 24°C X | 28°C X |
|---|---|---|---|---|
| Ethanol produit (%vol) | 13,05 | 12,35 | 14,35 | 13,60 |
| Sucres résiduels (g/L) | 38,10 | 45,60 | 8,10 | 20,40 |

Bien entendu, on peut procéder de manière similaire pour introgresser tout autre allèle d'intérêt dans une souche de levure cible, ou n'introgresser qu'un seul des allèles dans la souche donneuse.

Dans le cas où on souhaite sélectionner une souche de levures présentant déjà les allèles favorables, on supprime les étapes d'introgression, et on procède directement à la recherche des allèles d'intérêt au sein d'une population de levures donnée à l'aide de marqueurs moléculaires.

Il est également possible de procéder selon l'invention à une sélection de levures présentant un ou plusieurs allèles d'intérêt à partir de catalogues de souches mutantes générées par UV, EMS ou tout autre méthode de mutagenèse aléatoire.

### BIBLIOGRAPHIE

Berry, D, & Lindgren, B, (1995) Statistic, theory and methods. (International Thomson Publishing, ed.)
Chatonnet, P, Dubourdieu, D, Boidron, J & Lavigne, V (1993) Synthesis of volatile phenols by Saccharomyces cerevisiae in wines. J Sci Food Agric 62: 191-202.
Goodey, AR & Tubb, RS (1982) Genetic and biochemical analysis of the ability of Saccharomyces cerevisiae to decarboxylate cinnamic acid. J Gen Microbiol 128: 2615-2620.
Gresham, D., D. M. Ruderfer, S. C. Pratt, J. Schacherer, M. J. Dunham, D. Botstein, and L. Kruglyak. 2006. Genome-wide détection of polymorphisms at nucleotide resolution with a single DNA microarray. Science 311:1932-6.
Lander, ES & Botstein, D (1989) Mapping mendelian factors underlying quantitative traits using RFLP linkage maps. Genetics 121: 185-199.
Lynch M & Walsh B (1998) in Genetics and analysis of quantitative traits, (Sinauer Associates, Inc. eds).
Marullo, P, Bely, M, Masneuf-Pomarede, I, Aigle, M & Dubourdieu, D (2004) Inheritable nature of enological quantitative traits is demonstrated by meiotic segregation of industrial wine yeast strains. FEMS Yeast Research 4: 711-719.
Marullo, P, Bely, M, Masneuf-Pomarède, I, Pons, M, Aigle, M & Dubourdieu, D (2006) Breeding strategies for combining fermentative qualities and reducing Off-flavor production in a wine yeast model. FEMS Yeast Research 6: 268-279.
Marullo, P, Aigle, M, , Bely, M, Masneuf-Pomarède, I, Durrens, P, Dubourdieu, D & Yvert, G (2007a) Single nucleotide resolution QTL mapping of an enological trait from derived wild Saccharomyces cerevisiae strains. FEMS Yeast Research. 7: 941-952.
Marullo, P, Mansour, C Bely, M & Dubourdieu, D (2007b) Introgression o temperature resistance in S. Cerevisiae for red winemaking. Proceeding of Australian Wine industry Technical Conference, p264.
Romano, P, Soli, G, Suzzi, G, Grazia, L & Zambonelli, C (1985) Improvement of a wine Saccharomyces cerevisiae strain by a breeding program. Appl Environ Microbiol 50: 1064-1067.

## Revendications

1. Procédé de sélection de levures oenologiques de l'espèce *Saccharomyces cerevisiae* en fonction d'au moins un caractère oenologique, **caractérisé en ce qu'**il comporte les étapes suivantes :
- on identifie un locus associé à un caractère oenologique d'intérêt, lié à la production de phénols volatiles ;
- on identifie un allèle favorable dudit locus, associé à l'absence de production de phénols volatiles, ledit allèle favorable étant **caractérisé par** un polymorphisme de séquence avec le gène PAD1 sur la base 638 dudit gène PAD1, ledit polymorphisme étant une mutation de l'adénine en guanine entraînant la conversion de l'acide aspartique en glycine en position 213 de la protéine ;
- on réalise un marqueur moléculaire apte à détecter l'allèle favorable dudit locus ;
- on crible des levures oenologiques de l'espèce *Saccharomyces cerevisiae* avec le marqueur moléculaire de manière à sélectionner les levures possédant ledit allèle favorable et donc le caractère oenologiques d'intérêt.

2. Procédé de sélection selon la revendication 1, **caractérisé en ce qu'**il comporte les étapes suivantes :
- on amplifie un fragment d'ADN génomique des levures par réaction de polymérisation en chaîne (PCR) au moyen d'une amorce de séquence SEQ ID N° 8 et d'une amorce de séquence SEQ ID N° 9 ;
- on soumet ledit fragment à une digestion enzymatique par l'enzyme de restriction BgII ;
- on sélectionne des levures **caractérisées par** la présence d'une coupure par l'enzyme BgII, lesdites levures possédant ledit allèle favorable.

3. Procédé de sélection de levures oenologiques selon l'une des revendications 1 ou 2, **caractérisé en ce que** ledit allèle favorable a pour séquence SEQ ID N°7.

4. Procédé de sélection selon l'une quelconque des revendications 1 à 3, **caractérisé en ce qu'**il comporte les étapes suivantes :
- on sélectionne une souche de levure élite ;
- on sélectionne à l'aide de marqueurs moléculaires une souche de levures donneuses présentant au moins un allèle favorable associé au caractère oenologique d'intérêt,
- on introduit par introgression l'allèle favorable dans des descendants de la souche de levure élite
- on sélectionne à l'aide de marqueurs moléculaires des descendants de la souche de levure élite présentant l'allèle favorable.

5. Procédé de sélection selon la revendication 4, **caractérisé en ce qu'**au moins un allèle favorable introduit dans la souche de levures élites a pour séquence SEQ ID N°7.

6. Procédé de sélection selon l'une des revendications 1 à 5, **caractérisé en ce que** l'allèle favorable est présent sous forme homozygote dans les levures sélectionnées.

7. Procédé de sélection selon l'une des revendications 1 à 5, **caractérisé en ce que** l'allèle favorable est présent sous forme hétérozygote dans les levures sélectionnées.

8. Procédé de sélection selon l'une des revendications 1 à 7, **caractérisé en ce qu'**il comporte l'étape suivante :
- on sélectionne des levures oenologiques présentant un allèle selon SEQ ID N°4, lesdites levures sélectionnées ayant des performances améliorées pour la production de sulfure d'hydrogène.

9. Procédé de sélection selon l'une des revendications 1 à 8, **caractérisé en ce qu'**il comporte l'étape suivante :
- on sélectionne des levures oenologiques présentant un allèle selon SEQ ID N°19 et/ou SEQ ID N°16, lesdites levures sélectionnées ayant des performances améliorées pour la teneur en sucres résiduels en fin de fermentation alcoolique.

10. Procédé de sélection selon l'une des revendications 1 à 9, **caractérisé en ce qu'**il comporte l'étape suivante :
- on sélectionne des levures oenologiques présentant un allèle selon SEQ ID N°10 et/ou SEQ ID N°13, lesdites levures sélectionnées ayant des performances améliorées pour le temps pour dégager 80% de CO₂.

11. Procédé de sélection selon l'une des revendications 1 à 10, **caractérisé en ce qu'**il comporte l'étape suivante :
- on sélectionne des levures oenologiques présentant un allèle selon SEQ ID N°1, lesdites levures sélectionnées ayant des performances améliorées pour la vitesse relative à mi-fermentation, la durée de fermentation et la teneur en sucres résiduels en fin de fermentation alcoolique.

12. Séquence nucléotidique ayant la séquence nucléotidique de SEQ ID N°7.

13. Souche de levures *de l'espèce Saccharomyces cerevisiae* obtenue par un procédé selon la revendication 4, ne produisant pas de phénols volatiles.

## Patentansprüche

1. Verfahren zur Auswahl önologischer Hefen der Sorte *Saccharomyces cerevisiae* in Abhängigkeit von zumindest einem önologischen Merkmal, **dadurch gekennzeichnet, dass** es die folgenden Schritte umfasst:
- man identifiziert einen Ort, der einem önologischen Merkmal von Interesse zugeordnet wird, in Verbindung mit der Produktion flüchtiger Phenole;
- man identifiziert ein günstiges Allel des besagten Ortes, an dem keine Produktion flüchtiger Phenole stattfindet, wobei das besagte günstige Allel durch einen Polymorphismus der Sequenz mit dem PAD1 Gen auf der Basis 638 des besagten PAD1 Gens gekennzeichnet ist, und der besagte Polymorphismus eine Mutation des Adenins in Guanin ist, die zu einer Umwandlung der Asparaginsäure in Glycin in der Position 213 des Proteins führt;
- man erstellt einen Molekülmarker, der in der Lage ist, das günstige Allel des besagten Ortes zu erfassen;
- man sortiert die önologischen Hefen der Sorte *Saccharomyces cerevisiae* mit dem Molekülmarker, sodass jene Hefen ausgewählt werden, die das besagte günstige Allel, und somit das önologische Merkmal von Interesse besitzen.

2. Auswahlverfahren nach Anspruch 1, **dadurch gekennzeichnet, dass** es die folgenden Schritte umfasst:
- man verstärkt ein genomisches DNA-Fragment der Hefen durch eine Polymerase-Kettenreaktion (PCR) durch eine Primer einer Sequenz SEQ ID Nr.8 und eine Primer einer Sequenz SEQ ID Nr.9;
- man unterzieht das besagte Fragment einer enzymatischen Digestion durch die BgII Restriktionsendonuklease;
- man wählt die Hefen aus, die durch das Vorhandensein eines Schnitts durch die BgII Nuklease gekennzeichnet sind, wobei die besagten Hefen über das besagte günstige Allel verfügen.

3. Verfahren zur Auswahl önologischer Hefen nach einem der Ansprüche 1 oder 2, **dadurch gekennzeichnet, dass** das besagte günstige Allel die Sequenz SEQ ID Nr.7 aufweist.

4. Auswahlverfahren nach irgendeinem der Ansprüche 1 bis 3, **dadurch gekennzeichnet, dass** es die folgenden Schritte umfasst:
- man wählt einen Elite-Stamm einer Hefe aus;
- man wählt mithilfe von Molekülmarkern einen Stamm von Geberhefen aus, die zumindest ein günstiges Allel aufweisen, das dem önologischen Merkmal von Interesse zugeordnet ist,
- man führt das günstige Allel durch Introgression in die Abkömmlinge des Elite-Stamms einer Hefe ein
- man wählt mithilfe der Molekülmarker die Abkömmlinge des Elite-Stamms einer Hefe aus, die das günstige Allel aufweisen.

5. Auswahlverfahren nach Anspruch 4, **dadurch gekennzeichnet, dass** zumindest ein günstiges Allel, das in den Elite-Stamm einer Hefe eingeführt wird, eine Sequenz SEQ ID Nr.7 aufweist.

6. Auswahlverfahren nach irgendeinem der Ansprüche 1 bis 5, **dadurch gekennzeichnet, dass** das günstige Allel in den ausgewählten Hefen in homozygoter Form vorhanden ist.

7. Auswahlverfahren nach irgendeinem der Ansprüche 1 bis 5, **dadurch gekennzeichnet, dass** das günstige Allel in den ausgewählten Hefen in heterozygoter Form vorhanden ist.

8. Auswahlverfahren nach einem der Ansprüche 1 bis 7, **dadurch gekennzeichnet, dass** es den folgenden Schritt umfasst:
- man wählt önologische Hefen aus, die ein Allel gemäß der Sequenz SEQ ID Nr.4 aufweisen, und die besagten ausgewählten Hefen verbesserte Leistungen bei der Produktion von Schwefelwasserstoff aufweisen.

9. Auswahlverfahren nach einem der Ansprüche 1 bis 8, **dadurch gekennzeichnet, dass** es den folgenden Schritt umfasst:
- man wählt önologische Hefen aus, die ein Allel gemäß der Sequenz SEQ ID Nr.19 und/ oder SEQ ID Nr.16 aufweisen, und die besagten ausgewählten Hefen verbesserte Leistungen beim Gehalt an Restzucker am Ende der alkoholischen Gärung aufweisen.

10. Auswahlverfahren nach einem der Ansprüche 1 bis 9, **dadurch gekennzeichnet, dass** es den folgenden Schritt umfasst:
- man wählt önologische Hefen aus, die ein Allel gemäß der Sequenz SEQ ID Nr.10 und/ oder SEQ ID Nr.13 aufweisen, und die besagten ausgewählten Hefen verbesserte Leistungen bei der Zeit zum Ausbringen von 80% an CO₂ aufweisen.

11. Auswahlverfahren nach einem der Ansprüche 1 bis 10, **dadurch gekennzeichnet, dass** es den folgenden Schritt umfasst:
- man wählt önologische Hefen aus, die ein Allel gemäß der Sequenz SEQ ID Nr.1 aufweisen, und die besagten ausgewählten Hefen verbesserte Leistungen bei der relativen Geschwindigkeit bei Halbgärung, der Gärungsdauer und beim Gehalt an Restzucker am Ende der alkoholischen Gärung aufweisen.

12. Nukleotidsequenz mit der Nukleotidsequenz von SEQ ID Nr.7.

13. Hefestamm der Sorte *Saccharomyces cerevisiae,* die man durch ein Verfahren nach Anspruch 4 erhält, und die keine flüchtigen Phenole produziert.

## Claims

1. A method of selection of oenological yeasts of the species *Saccharomyces cerevisiae* based on at least one oenological character, **characterised in that** it comprises the following steps:
- identifying a locus associated with an oenological character of interest, linked with the production of phenolic-off flavor;
- identifying a suitable allele of said locus, associated with the absence of production of phenolic-off flavor, said suitable allele being **characterised by** a sequence polymorphism with respect to the PAD1 gene on base 638 of said PAD1 gene, said polymorphism being a mutation of adenine to guanine driving the conversion of aspartic acid to glycine in position 213 of the protein;
- making a molecular marker capable of detecting the suitable allele of said locus;
- screening oenological yeasts of the species *Saccharomyces cerevisiae* with the molecular marker in order to select the yeasts possessing said suitable allele and therefore the oenological character of interest.

2. A method of selection according to claim 1, **characterised in that** it comprises the following steps:
- amplifying a fragment of genomic DNA of the yeasts by polymerase chain reaction (PCR) by means of a primer having sequence SEQ ID No 8 and a primer having sequence SEQ ID No 9;
- subjecting said fragment to an enzymatic digestion by the Bgll restriction enzyme;
- selecting the yeasts **characterised by** the presence of a cutting by the Bgll enzyme, said yeasts possessing said suitable allele.

3. A method of selection of oenological yeasts according to one of claims 1 or 2, **characterised in that** said suitable allele has the sequence SEQ ID No7.

4. A method of selection according to any of claims 1 to 3, **characterised in that** it comprises the following steps:
- selecting an elite yeast strain;
- selecting by means of molecular markers a donor yeast strain presenting at least one suitable allele associated with the oenological character of interest;
- introducing the suitable allele by introgression into a progeny of the elite yeast strain;
- selecting by means of molecular markers progeny stains of the elite yeast strain having the suitable allele.

5. A method of selection according to claim 4, **characterised in that** at least one suitable allele introduced into the elite yeast strain has the sequence SEQ ID No7.

6. A method of selection according to one of claims 1 to 5, **characterised in that** the suitable allele is present in homozygous form in the selected yeasts.

7. A method of selection according to one of claims 1 to 5, **characterised in that** the suitable allele is present in heterozygous form in the selected yeasts.

8. A method of selection according to one of claims 1 to 7, **characterised in that** it comprises the following step:
- selecting oenological yeasts presenting an allele according to SEQ ID No4, said selected yeasts having improved performances for the production of hydrogen sulfide.

9. A method of selection according to one of claims 1 to 8, **characterised in that** it comprises the following step:
- selecting oenological yeasts presenting an allele according to SEQ ID No19 and/or SEQ ID No16, said selected yeasts having improved performances with respect to the content of residual sugars at the end of alcoholic fermentation.

10. A method of selection according to one of claims 1 to 9, **characterised in that** it comprises the following step:
- selecting oenological yeasts presenting an allele according to SEQ ID No10 and/or SEQ ID No13, said selected yeasts having improved performances with respect to the time for releasing 80% of CO₂.

11. A method of selection according to one of claims 1 to 10, **characterised in that** it comprises the following step:
- selecting oenological yeasts presenting an allele according to SEQ ID No1, said selected yeasts having improved performances with respect to the relative rate at half fermentation, the duration of fermentation and the content of residual sugars at the end of alcoholic fermentation.

12. A nucleotide sequence having the nucleotide sequence of SEQ ID No7.

13. A yeast strain *of the species Saccharomyces cerevisiae* obtained by a method according to claim 4, not producing phenolic-off flavor.


### SEQUENCE LISTING

<110> SARCO
<120> procédé de sélection de levures oenologiques
<130> 16631 WO
<150> FR 07/57245
   <151> 2007-08-29
<160> 21
<170> PatentIn version 3.3
<210> 1
   <211> 818
   <212> DNA
   <213> Saccharomyces cerevisiae
<400> 1
   tcatgttgac ctgaccatcc aagatctatt ggatgccatt ccagatatat ccaaggtctg 60
   tgacattgaa tatgagcaac tatgcaacgt ggattctaaa gacataaacg aggatattct 120
   ttataaaatt tataagggcg tctcagaatc gttgcaggct tttgacggta tagttattac 180
   ccatgggact catacgctat ctgaaactgc attctttatt gaaagtacta ttgatgctgg 240
   cgacgttcct attgtttttg ttggttcgat gcgtccttca accagcgttt ctgctgatgg 300
   ccctatgaac ctttaccaag caatttgcat tgcttcaaat ccaaaatcta gaggaagagg 360
   tgttcttgtt tccttgaatg accaaatttc ctctggttac tacattacta agacgaatgc 420
   aaatagtttg gattctttta atgttagaca aggctattta ggaaattttg tcaacaatga 480
   aattcactac tattatcctc ctgtgaaacc gcaaggttgc cacaaattca aactgagagt 540
   ggacggtaag cattttaaat taccagaggt ttgcatttta tatgctcacc aagcctttcc 600
   gccagctata gtcaacttag tggcagataa gtatgatggt attgttcttg ctaccatggg 660
   tgctggttca ttgccggagg aggtcaatga aacctgcatg aaattgagtt tgccgatcgt 720
   atattccaag agatcgatgg atggtatggt gcctattgcc aacgtaccaa agaaaggttc 780
   aaaggaggat aatctcatcg catctggtta tctaagcc 818
<210> 2
   <211> 21
   <212> DNA
   <213> Saccharomyces cerevisiae
<400> 2
   tcatgttgac ctgaccatcc a 21
<210> 3
   <211> 21
   <212> DNA
   <213> Saccharomyces cerevisiae
<400> 3
   ggcttagata accagatgcg a 21
<210> 4
   <211> 620
   <212> DNA
   <213> Saccharomyces cerevisiae
<400> 4
   gaaacgagtg ttcagttaca ggaggtttca ggaggtttac gagggctata aggtatatga     60
   aaagctatta caaaaatttg atgggcaaaa aaaaagaatg gagtctttct tgaatgaaaa    120
   cacgccaatg tcaggtgccg aagctatcaa gcaaataaat atatctgagg agctcaagga    180
   aaaaggggaa cgtttaacaa caccaaacga tccgttatta cacgttcagg tgagtaataa    240
   ggataactcg ctgcacttta tattgtacaa taaaacgaat atcataattc caggaaattg    300
   tacatttgaa ttttctagcc aaatctccga ggtattcagt atcaaaatgg gaccgaatga    360
   gataggcata aagggtcaga aagagctatg gtttttccca tcattaccta ctcctctctc    420
   caattacaca atgaaagtcg taaaccagga tggagatact attctggttg gtaaatgtgc    480
   tgactccaat gaaataactc taaaatctcc actagcctca ttttcgacag gcagtttcca    540
   gacaggcagt ttccacactt tgcaagatcc tactaatgtt ttccgggcag acgcgctttc    600
   ttccgctgat gaatcaagca                                                620
<210> 5
   <211> 21
   <212> DNA
   <213> Saccharomyces cerevisiae
<400> 5
   cgagtgttca gttacaggag g 21
<210> 6
   <211> 20
   <212> DNA
   <213> Saccharomyces cerevisiae
<400> 6
   tgcttgattc atcaggggaa 20
<210> 7
   <211> 813
   <212> DNA
   <213> Saccharomyces cerevisiae
<400> 7
   gcaaatatag attgatttca atctacggag tccaacgcat tgaacagctt caattgagta 60
   gatatgctcc tatttccaag aagaactaat atagcctttt tcaaaacaac aggcattttt 120
   gctaattttc ctttgctagg tagaaccatt acaacttcac catctttcct tacatataaa 180
   ctgtcaaagg aagtaaccag ggtatcaact tcgcctccaa gaccaaagag aattgttgtt 240
   gcaattactg gtgcgactgg tgttgcactg ggaatcagac ttctacaagt gctaaaagag 300
   ttgagcgtag aaacacattt ggtgatttca aaatggggtg cagcaacaat gaaatatgaa 360
   acagattggg aaccgcatga cgtggcggcc ttggcaacca agacatactc tgttcgcgat 420
   gtttctgcat gcatttcgtc cggatctttc cagcatgatg gtatgattgt tgtgccctgt 480
   tccatgaaat cactagctgc tattagaatt ggttttacag aggatttaat tacaagagct 540
   gccgatgttt cgattaaaga gaatcgtaag ttactactgg ttactcggga aaccccttta 600
   tcttccatcc atgttgaaaa catgttgtct ttatgcaggg caggtgttat aatttttcct 660
   ccggtacctg ctttttatac aagacccaag agccttcatg gcctattaga acaaagtgtt 720
   ggcaggatcc tagactgctt tggcatccac gctgacactt ttcctcgttg ggaaggaata 780
   aaaagcaagt aacacttttt ctgagcatta tag 813
<210> 8
   <211> 22
   <212> DNA
   <213> Saccharomyces cerevisiae
<400> 8
   gcaaatatag attgatttca at 22
<210> 9
   <211> 20
   <212> DNA
   <213> Saccharomyces cerevisiae
<400> 9
   ctataatgct cagaaaaagt 20
<210> 10
   <211> 47
   <212> DNA
   <213> Saccharomyces cerevisiae
<220>
   <221> misc_feature
   <222> (13)..(13)
   <223> n is a, c, g, or t
<400> 10
   ggttttgata ganttgtgca atctcaagaa atcaagaaca acaacca 47
<210> 11
   <211> 18
   <212> DNA
   <213> Saccharomyces cerevisiae
<400> 11
   cctaaacggg cttttgaa 18
<210> 12
   <211> 21
   <212> DNA
   <213> Saccharomyces cerevisiae
<400> 12
   aaggacgctg aaaaaagtaa a 21
<210> 13
   <211> 53
   <212> DNA
   <213> Saccharomyces cerevisiae
<400> 13
   agggaggcgg tcctattgga tttgcatcat catcaccgaa ggaatagggc tga 53
<210> 14
   <211> 21
   <212> DNA
   <213> Saccharomyces cerevisiae
<400> 14
   aatgtctcat ccaatttgtc g 21
<210> 15
   <211> 21
   <212> DNA
   <213> Saccharomyces cerevisiae
<400> 15
   cggtattgtt attggtgttg t 21
<210> 16
   <211> 60
   <212> DNA
   <213> Saccharomyces cerevisiae
<400> 16
   ttgaatagta aaagcatatc gaaatggata tggatacgca ggaggcagaa ctatcgagcc 60
<210> 17
   <211> 20
   <212> DNA
   <213> Saccharomyces cerevisiae
<400> 17
   gcttatgcgg gtagtgacct 20
<210> 18
   <211> 21
   <212> DNA
   <213> Saccharomyces cerevisiae
<400> 18
   tgcaccgtgc tatagttgtt c 21
<210> 19
   <211> 56
   <212> DNA
   <213> Saccharomyces cerevisiae
<400> 19
   aggcgagtga attaagaact ttcttcctgt attactctca ttcaatgcca gttcga 56
<210> 20
   <211> 19
   <212> DNA
   <213> Saccharomyces cerevisiae
<400> 20
   tgatatagaa aatgcgcaa 19
<210> 21
   <211> 20
   <212> DNA
   <213> Saccharomyces cerevisiae
<400> 21
   tactgcccgc cttatataca 20